(19) Europäisches Patentamt
European Patent Office
Office européen des brevets

(11) EP 2 915 797 A1

(12) EUROPÄISCHE PATENTANMELDUNG

(43) Veröffentlichungstag:
**09.09.2015 Patentblatt 2015/37**

(21) Anmeldenummer: **14158373.2**

(22) Anmeldetag: **07.03.2014**

(51) Int Cl.:
***C07C 45/29*** (2006.01)
***C07C 49/407*** (2006.01)
***A61Q 11/00*** (2006.01)
***A61K 8/35*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **BASF SE**
**67056 Ludwigshafen (DE)**

(72) Erfinder:
- **HICKMANN, Volker**
**67063 Ludwigshafen (DE)**
- **RÜDENAUER, Stefan**
**69469 Weinheim (DE)**
- **DEHN, Martine**
**67061 Ludwigshafen (DE)**
- **KELLER, Andreas**
**67346 Speyer (DE)**
- **RENZ, Stephanie**
**68723 Schwetzingen (DE)**
- **SCHNEIDER, Daniel**
**67227 Frankenthal (DE)**

(74) Vertreter: **Reitstötter Kinzebach**
**Patentanwälte**
**Sternwartstrasse 4**
**81679 München (DE)**

# (54) Verfahren zur Herstellung von Menthonen aus Isopulegol in der Gasphase

(57) Die vorliegende Erfindung betrifft ein Verfahren zur Umsetzung von Isopulegol zu Menthon in der Gasphase mit einem aktivierten Kupferkatalysator und die Verwendung der so hergestellten Reaktionsprodukte als Zusatz in Lebensmitteln, Kosmetika, pharmazeutischen Erzeugnissen, Tabakformulierungen, Haushaltsprodukten und Wäschepflegeprodukten.

EP 2 915 797 A1

**Beschreibung**

Die vorliegende Erfindung betrifft ein Verfahren zur Umsetzung von Isopulegol zu Menthon in der Gasphase und die Verwendung der so hergestellten Reaktionsprodukte als Zusatz in Lebensmitteln, Kosmetika, pharmazeutischen Erzeugnissen, Tabakformulierungen, Haushaltsprodukten und Wäschepflegeprodukten.

## Hintergrund der Erfindung

Menthol (1), der Hauptbestandteil der Kornminze (*Mentha avensis*), gehört zu den wichtigsten Aromastoffen der Riech- und Geschmackstoffindustrie und kann in Form von vier Diastereomeren vorliegen. Die oxidierten Diastereomeren des Menthols werden als Menthon (2) bzw. Isomenthon (3) bezeichnet. Im Falle der Diastereomeren von Menthon liegen die beiden Alkylsubstituenten *trans*-ständig vor, bei Isomenthon sind die Substituenten cis-konfiguriert.

Menthon findet aufgrund seines an Pfefferminze erinnernden Geruchs- und Geschmacksprofils in vielen Formulierungen Einsatz, beispielsweise für Mundpflege und Kaugummi-Anwendungen.

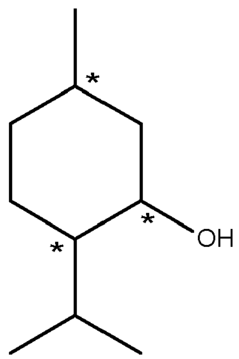
Menthol (1)

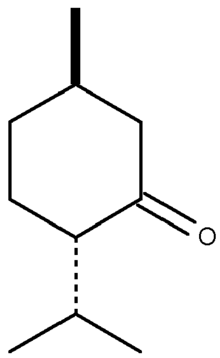
Menthon (2)

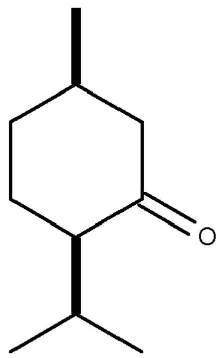
Isomenthon (3)

Üblicherweise wird Menthon oxidativ ausgehend von Menthol gewonnen. Dabei verwendete Oxidationsmittel sind Natriumchromat/Schwefelsäure (Spec. Chem. 1987, 193; Acta Chem. Scand. B 1979, 148), Natriumhypochlorit/Essigsäure *(*J. Org. Chem. 1980, 2030), Ozon/Ethylacetat (JP 82180463) oder Pyridiniumchlorochromat/Silicagel *(*Tetrahedron 1979, 1789). In der Summe sind die oxidativen Methoden zur Menthonherstellung aufgrund der Verwendung hinsichtlich des Arbeitsschutzes und der Umweltverträglichkeit bedenklicher Reagenzien nicht als befriedigend anzusehen.

Die DE 4236111 A1 beschreibt ein Verfahren zur Herstellung von Menthon aus Menthol im Festbettreaktor unter Verwendung eines heterogenen Dehydrierungskatalysators auf Kupferbasis.

W. Treibs et al. Chem. Ber. 1927, 2335 beschreiben die Umsetzung von Isopulegol (4) zu Menthon (2) in Gegenwart eines Katalysators, der aus Kupferacetat durch Fällung mit NaOH gewonnen wird.

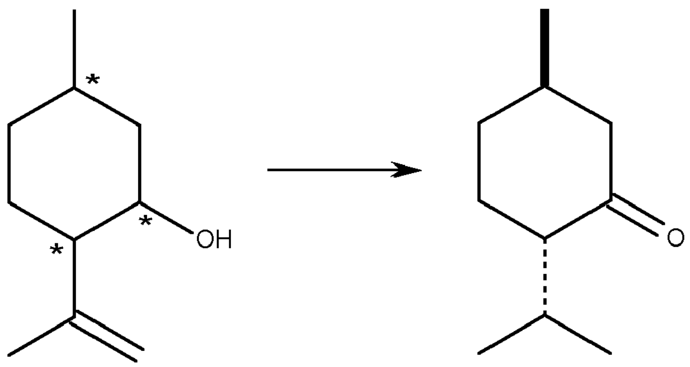
Isopulegol (4) Menthon (2)

Bei einer Reaktionstemperatur von 280 °C kann nach Überleiten des Ausgangsstoffes über den Katalysator ein Gemisch aus Menthon, Isomenthon und Thymol isoliert werden. Die darin offenbarte Reaktionsführung wird vor allem aufgrund der umständlichen Herstellung des Katalysators und der hohen Temperaturen, die die Bildung von Nebenprodukten begünstigen, als nachteilig betrachtet.

Eine weitere Möglichkeit zur Menthonherstellung, in diesem Fall ausgehend von Citronellol (5), wird in der US 4,134,919 beschrieben.

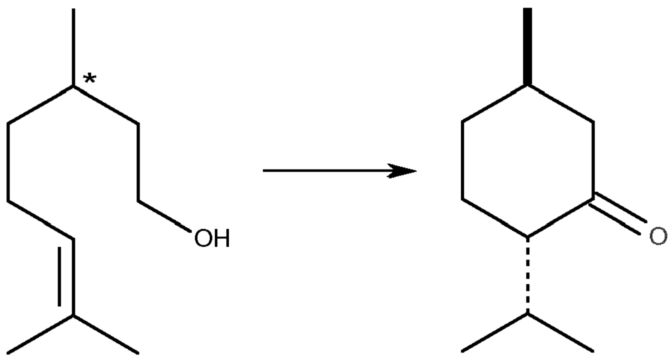

Citronellol (5) Menthon (2)

Dabei wird innerhalb von vier Stunden bei Temperaturen von 150-260 °C und kontinuierlichem Wasserstoffdruck Citronellol zu einem Menthon-/Isomenthongemisch umgesetzt. Als Katalysatoren werden Verbindungen auf Kupferbasis, z.B. Cu/Cr, Cu/Al oder Cu/Zn, eingesetzt. Isomenthon und Menthon können auch in enantiomerenangereicherter Form erhalten werden. Dabei werden Enantiomerenüberschüsse von maximal 80% erreicht.

Auch Citronellal (6) kann als Ausgangsstoff für die Menthonherstellung dienen. Forti et al. Synthesis 2001, 1, 52 beschreiben die Cyclisierung von Citronellal in Gegenwart von Calcium-Schichtsilikaten, Aluminium- und Eisennitrat sowie einer NaOH-Lösung in Dichlorethan. Das Produktgemisch enthält Menthon und Isomenthon in einem Verhältnis von 69/31.

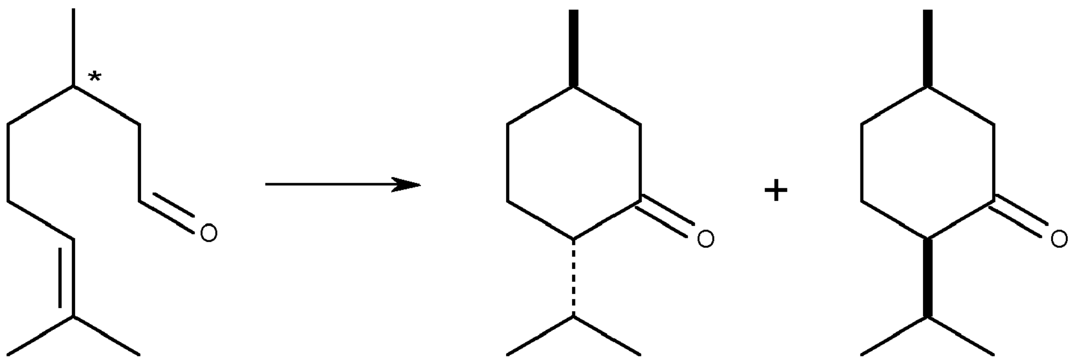

Citronellal (6) Menthon (2) Isomenthon (3)

Zusammenfassend ist festzustellen, dass die aus dem Stand der Technik bekannten Verfahren die Synthese von Menthon, auch in enantiomerenangereicherter Form, ermöglichen, jedoch die Verwendung von umwelt- oder gesundheitsschädlichen Reagenzien beinhalten. Weiterhin liefern die offenbarten Verfahren lediglich Enantiomerenüberschüsse von maximal 80%. Ferner stellen die Durchführung der Reaktion vor allem in flüssigen Reaktionsmedien und die Verwendung schwer zugänglicher Katalysatoren wesentliche Nachteile dar.

Aufgabe der Erfindung ist somit die Bereitstellung eines verbesserten Verfahrens zur katalysierten Herstellung von Menthon, bei dem insbesondere Menthon mit einem erhöhten Anteil eines Enantiomers, insbesondere enantiomerenrein, erhalten wird.

## Kurzfassung der Erfindung

Diese Aufgabe wurde durch das erfindungsgemäße Verfahren gemäß Anspruch 1 gelöst, insbesondere durch Umsetzung von Isopulegol, mit einem aktivierten Kupferkatalysator und vor allem von Isopulegol, das einen erhöhten Anteil des Enantiomers mit (*R*)-Konfiguration in Position 5 aufweist, wobei Isopulegol in der Gasphase mit einem Kupferkatalysator in Kontakt gebracht wird. Dabei wird vor der Umsetzung von Isopulegol der genannte Kupferkatalysator mit Wasserstoff oder Wasserstoff und einem Alkohol (gleichzeitig oder zeitlich versetzt in beliebiger Reihenfolge), gegebenenfalls in einem Trägergasstrom, in Kontakt gebracht. Nach der Umsetzung wird das Reaktionsprodukt gegebenenfalls isoliert.

Ein wesentliches Produkt der Umsetzung ist Menthon, das insbesondere einen erhöhten Anteil des Enantiomers mit (*R*)-Konfiguration in Position 5 aufweist.

## Detaillierte Beschreibung der Erfindung

### a) Allgemeine Definitionen

Werden keine gegenteiligen Angaben gemacht, so umfasst "Isopulegol" die möglichen Stereoisomere:

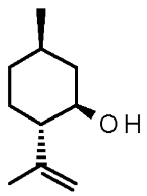

(1*R*,2*S*,5*R*)-(-)-Isopulegol

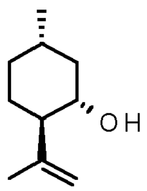

(1*S*,2*R*,5*S*)-(+)-Isopulegol

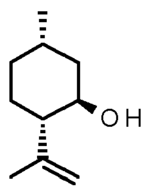

(1*R*,2*S*,5*S*)-2-Isopropenyl-5-methylcyclohexanol

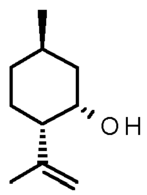

(1*S*,2*S*,5*R*)-2-Isopropenyl-5-methylcyclohexanol

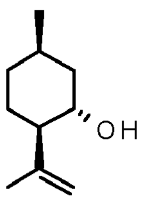

(1*S*,2*R*,5*R*)-2-Isopropenyl-5-methylcyclohexanol

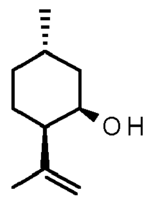

(1*R*,2*R*,5*S*)-2-Isopropenyl-5-methylcyclohexanol

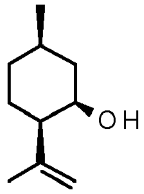 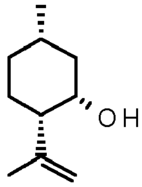

(1*R*,2*R*,5*R*)-2-Isopropenyl-5-methylcyclohexanol (1*S*,2*S*,5*S*)-2-Isopropenyl-5-methylcyclohexanol

Werden keine gegenteiligen Angaben gemacht, so umfasst "Menthon" die beiden Stereoisomere:

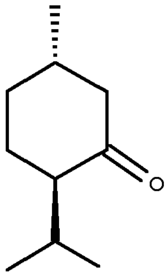 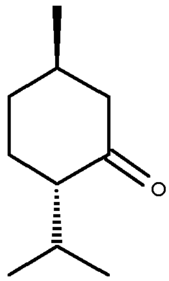

(+)-Menthon (-)-Menthon

Werden keine gegenteiligen Angaben gemacht, so umfasst "Isomenthon" die beiden Stereoisomere:

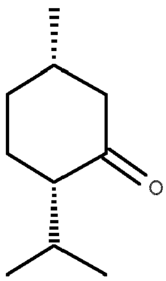 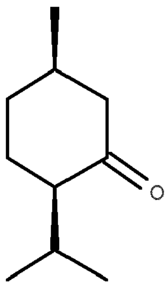

(-)-Isomenthon (+)-Isomenthon

"Enantiomerenrein" bedeutet, dass neben dem speziell benannten Enantiomer keine weitere enantiomere Form derselben chemischen Verbindung mit wenigstens einem Asymmetriezentrum analytisch nachweisbar ist.

"Enantiomerenüberschuss" gibt den Überschuss eines Enantiomers in einem Enantiomerengemisch an und wird nach folgender Formel berechnet:

$$ee = [\left| m_1 - m_2 \right| / (m_1 + m_2)] \times 100\%$$

ee: Enantiomerenüberschuss
$m_1$ Anteil des Enantiomers 1
$m_2$: Anteil des Enantiomers 2

"Monoole" umfassen Alkanole, i.e. Alkylalkohole, und Alkenole, i.e. Alkenylalkohole, mit einer Hydroxylgruppe.

"Polyole" umfassen mehrwertige Analoga von obigen Monoolen, d.h. Alkanole, i.e. Alkylalkohole, und Alkenole, i.e. Alkenylalkohole, mit mindestens zwei, insbesondere aber mehr als zwei Hydroxylgruppen. Diole umfassen insbesondere Alkandiole, i.e. zweiwertige Analoga von Alkylalkoholen, und Alkendiole, i.e. zweiwertige Analoga von Alkenylalkoholen. Dabei sind "Alkyl" und "Alkenyl" wie unten angegeben definiert.

"Alkyl" (bzw. "Alkan-" im Kontext von Alkan-Mono-oder -Polyolen) steht insbesondere für gesättigte, geradkettige oder verzweigte oder cyclische Kohlenwasserstoffreste mit 1 bis 20, 3 bis 16 oder 4 bis 12 Kohlenstoffatomen, wie z. B. Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, n-Octyl, n-Nonyl und n-Decyl, n-Dodecyl, n-Tetradecyl, n-Hexadecyl, n-Octadecyl, n-Eicosyl, Cyclobutyl, Cyclopentyl, Cyclohexyl, Cycloheptyl, Cyclooctyl, Cyclononyl, Cyclodecyl, Cyclododecyl, Cyclotetracyl, Cyclohexadecyl, Cyclooctadecyl, Cycloeicosyl, sowie die ein- oder mehrfach verzweigten Analoga davon.

"Alkenyl" (bzw. "Alken-" im Kontext von Alken-Mono- oder -Polyolen) steht insbesondere für die ungesättigten, geradkettigen, verzweigten oder cyclischen Analoga obiger Alkylreste und weist insbesondere 2 bis 20, 3 bis 16 oder 4 bis 12 Kohlenstoffatome auf. Insbesondere können diese ein- oder mehrfach, wie z.B. 2-, 3-, 4- oder 5-fach ungesättigt sein. Die Doppelbindungen können dabei kumuliert, konjugiert oder nichtkonjugiert vorliegen.

Ein "Kupferkatalysator" umfasst Zusammensetzungen, die Kupfer enthalten und für die Katalyse und Aktivierung der erfindungsgemäßen Gasphasenreaktion (Umsetzung von Isopulegol zu Menthon) geeignet sind. Kupfer kann darin insbesondere als Oxid vorliegen. Insbesondere kann Kupfer in den Oxidationsstufen +I und +II vorliegen. Neben Kupfer können weiterhin eines oder mehrere der Elemente Aluminium, Mangan, Barium, Chrom, Calcium oder Eisen in der Katalysatorzusammensetzung vorliegen. Insbesondere können diese Elemente elementar oder als Oxide, z.B. Kupferoxid, Aluminiumoxid, Manganoxid, Bariumoxid, Chromoxid oder Eisenoxid vorliegen.

Die Elemente Aluminium, Mangan, Barium, Chrom, Calcium oder Eisen können insbesondere in den Oxidationsstufen + I bis +VI vorliegen. Weiterhin können Kombinationen der Elemente Kupfer, Aluminium, Mangan, Barium, Chrom, Calcium oder Eisen als "Doppel"- oder "Mehrfachoxide", z.B. Dichromkupfertetraoxid, Aluminiumkupferoxid ($Al_2CuO_4$), Kupferchromat, Bariumchromat, Calciumsilikat oder Palygorskit, in der Katalysatorzusammensetzung vorliegen. In den Doppel- oder Mehrfachoxiden liegen die genannten Elemente insbesondere in den Oxidationsstufen +I bis +VI vor.

Der "Kupferkatalysator" kann geträgert oder nicht geträgert vorliegen. Trägermaterialien sind z.B. Quarz, Siliciumdioxid, Aluminiumoxid oder Graphit.

Der Anteil an Kupferverbindungen im Katalysator beträgt mindestens 20 bis zu 100% bezogen auf das Gesamtgewicht des trockenen Katalysators. Insbesondere beträgt der Anteil an Kupferverbindungen im Katalysator 25 bis 95%, zum Beispiel 30-65%, insbesondere 30-45%, bezogen auf das Gesamtgewicht des trockenen Katalysators.

**b) Spezielle Ausgestaltungen**

Die vorliegende Erfindung betrifft insbesondere folgende Ausführungsformen:

1. Verfahren zur Umsetzung von Isopulegol, wobei Isopulegol in der Gasphase mit einem aktivierten Kupferkatalysator in Kontakt gebracht wird und gegebenenfalls nach der Umsetzung ein Menthon-haltiges Reaktionsprodukt isoliert wird.

2. Verfahren nach Ausführungsform 1, wobei der Kupferkatalysator mit Wasserstoff oder mit Wasserstoff und einem Alkohol aktiviert wird.

3. Verfahren nach einer der vorhergehenden Ausführungsformen, wobei das eingesetzte Isopulegol ein Enantiomer der Formel I umfasst, das durch (*R*)-Konfiguration in Position 5 gekennzeichnet ist.

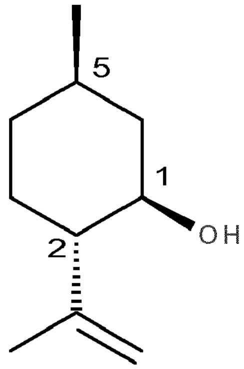

I

4. Verfahren nach einer der vorhergehenden Ausführungsformen, wobei die Reaktion bei einer Temperatur von 150-250 °C, insbesondere 160-200 °C, z.B. 170 °C, durchgeführt wird. Das Verfahren kann dabei bei 50 mBar bis 1000 mBar oder bis 1 Bar Überdruck betrieben werden. Bevorzugte Vakuumfahrweise sind 500 mBar, noch bevorzugter ist die Fahrweise bei Normaldruck.

5. Verfahren nach einer der vorhergehenden Ausführungsformen, wobei Isopulegol mit einem Katalysator auf Kupferbasis in Kontakt gebracht wird.

Dieser Kupferkatalysator umfasst Zusammensetzungen, die Kupfer enthalten und für die Katalyse und Aktivierung der erfindungsgemäßen Gasphasenreaktion geeignet sind. Kupfer kann darin insbesondere als Oxid vorliegen. Insbesondere kann Kupfer in den Oxidationsstufen +I und +II vorliegen.

Neben Kupfer können weiterhin ein oder mehrere Elemente ausgewählt unter Aluminium, Mangan, Barium, Chrom, Calcium und Eisen in der Katalysatorzusammensetzung vorliegen. Insbesondere können diese Elemente elementar oder als Oxide, Aluminiumoxid, Manganoxid, Bariumoxid, Chromoxid oder Eisenoxid, z.B. zusammen mit Kupferoxid vorliegen.

Die Elemente Aluminium, Mangan, Barium, Chrom, Silicium, Calcium oder Eisen können insbesondere in den Oxidationsstufen + I bis +VI vorliegen.

Weiterhin können Kombinationen der Elemente Kupfer, Aluminium, Mangan, Barium, Chrom, Calcium oder Eisen als Doppel- oder Mehrfachoxide, z.B. Dichromkupfertetraoxid, Aluminiumkupferoxid ($Al_2CuO_4$), Kupferchromat, Bariumchromat oder Palygorskit, in der Katalysatorzusammensetzung vorliegen. In den Doppel- oder Mehrfachoxiden liegen die genannten Elemente insbesondere in den Oxidationsstufen +I bis +VI vor.

Der Kupferkatalysator kann geträgert oder nicht geträgert vorliegen. Trägermaterialien sind z.B. Quartz, Siliciumdioxid, Aluminiumoxid oder Graphit.

Der Anteil an Kupferverbindungen im Katalysator beträgt mindestens 20 bis zu 100% bezogen auf das Gesamtgewicht des trockenen Katalysators. Insbesondere beträgt der Anteil an Kupferverbindungen im Katalysator 25 bis 95%, z.B. 30-65%, insbesondere 30-45%, bezogen auf das Gesamtgewicht des trockenen Katalysators.

Beispiele für derartige Kupferkatalysatoren umfassen die Handelsprodukte X 540 T 1/8, E 406 T 1/8, Cu 1986 T 1/8, Cu 1808 T 1/8, Cu 1230 E1/16 oder Cu 0865 T 3/16 der BASF Corporation (Florham Park, NJ 07932, USA) mit den folgenden Zusammensetzungen:

**X 540 T 1/8***

30.0-45.0 Gew.-% Kupferoxid
30.0-40.0 Gew.-% Aluminiumkupferoxid ($Al_2CuO_4$)
10.0-25.0 Gew.-% Aluminumoxid
10.0-20.0 Gew.-% Mangandioxid

**E 406 T 1/8***

60.0-65.0 Gew.-% Chromkupferoxid ($Cr_2CuO_4$)
20.0-25.0 Gew.-% Kupferoxid
5.0-10.0 Gew.-% Bariumoxid
1.0-5.0 Gew.-% Graphit

1.0 Gew.-% Dichromtrioxid
1.0 Gew.-% Chromtrioxid

**Cu 1986 T 1/8***

60.0-70.0 Gew.-% Chromkupferoxid ($Cr_2CuO_4$)
20.0-30.0 Gew.-% Kupferoxid
1.0-5.0 Gew.-% Mangandioxid
1.0-5.0 Gew.-% Kieselsäure, Natriumsalz
1.0-5.0 Gew.-% Graphit
0.0-0.5 Gew.-% Kupferchromat

**Cu 1808 T 1/8***

55.0-65.0 Gew.-% Chromkupferoxid ($Cr_2CuO_4$)
20.0-30.0 Gew.-% Kupferoxid
5.0-10.0 Gew.-% Siliciumdioxid
5.0-10.0 Gew.-% Kieselsäure, Natriumsalz
1.0-5.0 Gew.-% Graphit

**Cu 1230 E 1/16***

41.0-46.0 Gew.-% Chromkupferoxid ($Cr_2CuO_4$)
25.0-35.0 Gew.-% Aluminumoxid
13.0-17.0 Gew.-% Kupferoxid
10.0-13.0 Gew.-% Bariumchromat

**Cu 0865 T 3/16***

55.0-65.0 Gew.-% Kupferoxid
25.0-35.0 Gew.-% Calciumsilikat
5.0-10.0 Gew.-% Palygorskit ($[Mg(Al_{0.5}\text{-}1Fe_{0\text{-}0.5})]Si_4(OH)O_{10.4}H_2O$)
1.0-5.0 Gew.-% Graphit
1.0-5.0 Gew.-% Siliciumdioxid
0.5-1.5 Gew.-% Silica (kristallin).

*alle Angaben bezogen auf das Gesamtgewicht des trockenen Katalysators.

6. Verfahren nach einer der vorhergehenden Ausführungsformen, wobei der Katalysator als homogener oder heterogener Katalysator eingesetzt wird. Insbesondere die heterogene Katalyse der Umsetzung von Isopulegol im Festbettreaktor stellt eine bevorzugte Ausführungsform der Erfindung dar.

7. Verfahren nach einer der vorhergehenden Ausführungsformen, wobei der Katalysator vor der Umsetzung mit Wasserstoff oder Wasserstoff und einem Alkohol (gleichzeitig oder zeitlich versetzt in beliebiger Reihenfolge), gegebenenfalls in einem Trägergasstrom, aktiviert wird.

Die Temperatur für die Aktivierung kann zwischen 150 und 220 °C liegen.

Eine bevorzugte Ausführungsform des Verfahrens ist die Aktivierung des Katalysators zunächst durch einen Wasserstoffstrom, der insbesondere unterstützt durch einen Trägergasstrom durch den Reaktor strömt. In einem zeitlich versetzten Verfahrensschritt erfolgt in dieser bevorzugten Ausführungsform nach der reduktiven Aktivierung die Aktivierung des Katalysators durch einen Alkohol, der mit oder ohne, insbesondere ohne Trägergasunterstützung durch den Reaktor geführt wird.

Eine weitere bevorzugte Ausführungsform des Verfahrens umfasst die zeitlich versetzte Aktivierung des Katalysators, zunächst durch Wasserstoff, danach durch einen Alkohol, wobei in keinem der Aktivierungsschritte Trägergas zugesetzt wird.

Eine weitere bevorzugte Ausführungsform des Verfahrens umfasst die Aktivierung des Katalysators zunächst durch Wasserstoff, danach durch Alkohol, insbesondere unter Zusatz von Trägergas in beiden Aktivierungsschritten.

Weitere spezielle Ausgestaltungen des erfindungsgemäßen Verfahrens umfassen die zeitlich versetzte Aktivierung des Katalysators zuerst durch einen Alkohol, danach durch Wasserstoff. Beispielsweise kann in einem, in beiden oder in keinem der Aktivierungsschritte Trägergas zugesetzt werden.

Ferner ist die gleichzeitige Aktivierung des Katalysators mit Wasserstoff und Alkohol eine weitere Ausführungsform des erfindungsgemäßen Verfahrens. Dabei kann die Aktivierung beispielsweise unterstützt durch Trägergasstrom oder ohne Trägergasstrom durchgeführt werden.

Die wie oben beschrieben aktivierten Katalysatoren können unter geeigneten Lösungsmitteln, insbesondere aliphatischen linearen oder verzweigten Alkoholen, vorzugsweise Isononanol, aufbewahrt werden.

8. Verfahren nach einer der vorhergehenden Ausführungsformen, wobei als Trägergas Stickstoff oder Argon oder Gemische davon verwendet werden. Dabei ist insbesondere die Verwendung von Stickstoff eine bevorzugte Ausführungsform des Verfahrens. Insbesondere das Trägergas-basierte Durchströmen des Reaktors während der erfindungsgemäßen Umsetzung und während der Aktivierung des Katalysators stellt eine bevorzugte Ausführungsform des erfindungsgemäßen Verfahrens dar. Weiterhin kann der Trägergasstrom während der Umsetzung bis zu 10 Vol.-% , wie z.B. 0,1 bis 10 Vol.-% Wasserstoff enthalten.

9. Verfahren nach Ausführungsform 8, wobei der Reaktor mit einer Rate von 0 bis 5 NL/h Trägergas pro g/h Substrat, insbesondere 0,5 bis 1,5 NL/h Trägergas pro g/h Substrat, durchströmt wird.

10. Verfahren nach einer der vorhergehenden Ausführungsformen, wobei der für die Aktivierung verwendete Alkohol ausgewählt ist unter gesättigten oder ein- oder mehrfach ungesättigten, geradkettigen oder verzweigten oder cyclischen aliphatischen oder aromatischen Mono- oder Polyolen, insbesondere Mono- oder Diolen, oder Kombinationen davon.

Insbesondere die Verwendung von Alkoholen mit $C_1$-$C_{20}$,-, $C_3$-$C_{16}$- oder $C_4$-$C_{12}$ Kohlenstoffatomen stellt eine bevorzugte Ausführungsform der Erfindung dar. Darunter sind insbesondere Alkohole, bei denen unter den Reaktionsbedingungen nicht mit einer Kondensation zu rechnen ist, z.B. 1,4-Butandiol, Hexanol, Cyclohexanol, Octanol, 1-Nonanol und Citronellol, eine bevorzugte Ausführungsform der Erfindung. Weiterhin stellen Alkohole, deren Verwendung die Bewahrung der Konfiguration des Stereozentrums an Position 5 von Isopulegol begünstigt, wie insbesondere 1-Nonanol, 1,4-Butandiol und Citronellol, eine bevorzugte Ausführungsform der Erfindung dar.

11. Verfahren nach einer der vorhergehenden Ausführungsformen, wobei die Umsetzung von Isopulegol in einer Gasphasenapparatur mit gekoppeltem Verdampfer durchgeführt wird. Dabei ist der Verdampfer beispielsweise oberhalb des Reaktors angebracht und dient dazu, die flüssigen Reaktionskomponenten, insbesondere Isopulegol und den gegebenenfalls zur Aktivierung verwendeten Alkohol, zu verdampfen und die eingesetzten Gasströme, z.B. Wasserstoff, Stickstoff oder Argon, gegebenenfalls, falls nicht bereits vorgeheizt, aufzuheizen.

12. Verfahren nach einer der vorhergehenden Ausführungsformen, wobei das Umsetzungsprodukt Menthon (2) und/oder Isomenthon (3) umfasst.

In einer bevorzugten Ausführungsform der Erfindung sind Menthon und Isomenthon die Hauptprodukte, als Nebenprodukte fallen beispielsweise Menthol (1) und Thymol (7) an. Insbesondere die Umsetzung zu Menthon und Isomenthon mit (*R*)-Konfiguration des Stereozentrums in Position 5 ist eine bevorzugte Ausführungsform des erfindungsgemäßen Verfahrens.

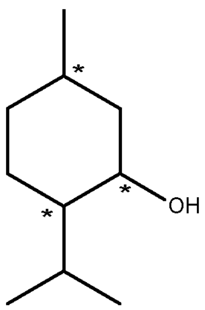
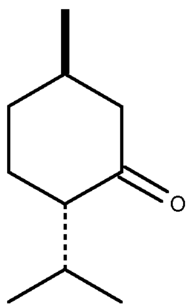
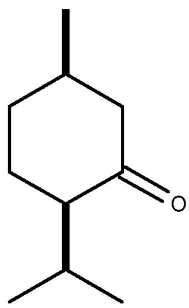
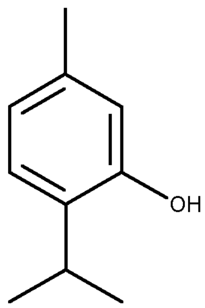

Menthol (1) Menthon (2) Isomenthon (3) Thymol (7)

13. Verfahren nach einer der vorhergehenden Ausführungsformen, wobei im Umsetzungsprodukt Menthon (8) und/oder Isomenthon (9) im Wesentlichen enantiomerenrein vorliegt.

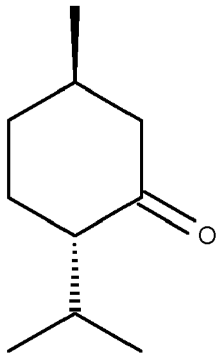
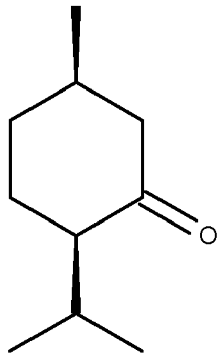

Menthon (8) Isomenthon (9)

14. Verwendung eines nach einem der vorhergehenden Ausführungsformen hergestellten Reaktionsprodukts als Zusatz in Lebensmitteln, Kosmetika, pharmazeutischen Erzeugnissen, Tabakformulierungen, Haushaltsprodukten und Wäschepflegeprodukten.

**c) Detaillierte Beschreibung des erfindungsgemäßen Verfahrens**

Anhand einer bevorzugten Ausführungsform zur Umsetzung von Isopulegol wird das erfindungsgemäße Verfahrensprinzip im Folgenden näher erläutert.

Die Gasphasenapparatur umfasst einen Reaktor, der beispielsweise ein oder mehrere Rohre beinhaltet, die entweder elektrisch, mit Wärmeträgerflüssigkeit oder mit heißen Gasen (z.B. Rauchgas) beheizt werden können. Der Festbettkatalysator liegt im Reaktor, beispielsweise in eines der Rohre gefüllt, vor. Im Katalysatorbett kann die Reaktionstemperatur gemessen werden. Das mit Substrat beladene Trägergas durchströmt das Katalysatorbett, zum Beispiel von oben nach unten. Gekoppelt an den Reaktor befindet sich ein Verdampfer, der beispielsweise oberhalb des Reaktors angebracht ist und durch dessen Verwendung die (flüssigen) Reaktanden in die Gasphase überführt werden können, ggf. unterstützt durch Einsatz von Trägergas. Auch der Trägergasstrom kann dann mittels des Verdampfers oder mittels eines separaten Vorheizers auf die Reaktionstemperatur aufgeheizt werden. Die Reaktionsprodukte kondensieren nach Austritt aus dem Reaktor entweder spontan, durch Quench mit kaltem Gas oder Flüssigkeit oder durch Kondensation an gekühlten Flächen. Der Übergang zwischen Verdampfer und Reaktor ist zum Zwecke der Vermeidung von Kondensation gut isoliert oder gar schutzbeheizt (zum Beispiel mit einem Heizband umwickelt).

In einer speziellen Ausführungsform wird der Gasphasenreaktor beispielsweise mit einem Kupferkatalysator, z.B. X540T 1/8, gefüllt und der Katalysator unter $H_2$-haltigem Gasstrom aktiviert. Die Temperatur für die Aktivierung kann zwischen 150 und 220 °C liegen. Verdampfer und Reaktor werden beispielsweise bei einer Temperatur von 150-220 °C bei 50 mBar bis 1000 mBar oder bis 1 Bar Überdruck betrieben. Bevorzugte Vakuumfahrweise sind 500 mBar, noch bevorzugter ist die Fahrweise bei Normaldruck, und unter Verwendung von Trägergas, wie zum Beispiel Stickstoff oder Argon und gegebenenfalls in Gegenwart von bis zu 10 Vol.-% Wasserstoff.,. Isopulegol wird kontinuierlich in den Verdampfer eingebracht. Das Produktgemisch wird am Reaktorausgang kondensiert und die Zusammensetzung gaschromatographisch analysiert.

Nach Umsetzung (z.B. nach 1 bis 20 , 2 bis 11 oder etwa 5 Stunden) werden Reaktor und Verdampfer unter Trägergasstrom abgekühlt und die Umsetzung, z.B. am Folgetag, ohne Katalysatorwechsel für einen oder mehrere Reaktionszyklen fortgesetzt. Nach jeweils definierten Reaktionszeiten, zum Beispiel eine Stunde, werden Umsatz, Massenbilanz und Produktzusammensetzung über Gaschromatographie bestimmt. Die detektierten Produkte umfassen beispielsweise Menthon, Isomenthon, Thymol und Menthol.

Wird bei der Ausführung des erfindungsgemäßen Verfahrens beispielsweise enantiomerenangereichertes Isopulegol als Ausgangsmaterial verwendet, kann weiterhin ein Menthon-/Isomenthongemisch in enantiomerenangereicherter Form detektiert werden. Der Enantiomerenüberschuss kann über chirale Gaschromatographie bestimmt werden. Dabei ist für das Erreichen eines hohen Enantiomerenüberschusses insbesondere bevorzugt das Katalysatorsystem nach der ersten reduktiven Aktivierung durch Wasserstoff noch ein zweites Mal durch die Verwendung eines Alkohols, beispielsweise 1-Nonanol-, 1,4-Butandiol- oder Citronellol, zu aktivieren.

Bei der Verwendung von Citronellol können z.B. Enantiomerenüberschüsse größer als 99% erreicht werden, für 1-Nonanol Werte von bis zu 98.4% und für 1,4-Butandiol Werte von bis zu 93.4%. In den genannten Umsetzungen ist beispielsweise das Enantiomer mit (*R*)-Konfiguration in Position 5 das bevorzugte.

Die Erfindung wird nun unter Bezugnahme auf folgende nichtlimitierende Beispiele näher erläutert.

## Experimenteller Teil

### A) Allgemeine Arbeitsmethoden

Die nachfolgenden Umsetzungen wurden in einer Gasphasenapparatur, umfassend einen Reaktor mit doppelwandigem Glasrohr, dessen inneres Rohr elektrisch beheizt werden kann und am unteren Ende eine Lochplatte enthält, durchgeführt. Der Reaktor wird mit dem Katalysator befüllt, dessen Aktivierung reduktiv durch einen $H_2$-haltigen Gasstrom (20-40 NL/h) bei einer Temperatur von 170-180 °C durchgeführt wird. Nach der Aktivierung des Katalysators wird der Reaktor unter Inertgasatmosphäre betrieben, das Trägergas Stickstoff strömt von oben nach unten durch den Reaktor. Die flüssigen Ausgangsmaterialien werden unter Verwendung eines Verdampfers, der analog dem Reaktor aufgebaut ist und oberhalb des Reaktors angebracht ist, in die Gasphase überführt. Die Kondensation des Produktgemisches erfolgt durch Wasserkühlung.

Die angegebenen Selektivitäten, Umsätze und Massenbilanzen wurden gaschromatographisch bestimmt und unter Verwendung eines Agilent 7890 A Gaschromatographen durchgeführt. Enantiomerenüberschüsse wurden mittels chiraler Gaschromatographie unter Verwendung eines Agilent 7890 A Gaschromatographen ermittelt.

### Gaschromatographie

| | |
|---|---|
| Gerät | : Agilent 7890 A |
| Säule | : 50 m CP-Wax, Innendurchmesser 0,32 mm, Filmdicke 1,2 μm |
| Eluent | : Stickstoff |
| Detektor | : FID, 250 °C |
| Injektion | : 0,2 μL, Split 100:1 |
| Temperatur | : Start 130 °C, 3 °C/min auf 150 °C, 20 min isotherm bei 150 °C, 10 °C/min auf 240 °C |
| Laufzeit | : 60 min |
| Druck | : 46,671 kPa (Druck geregelt) |

### Chirale Gaschromatographie

| | |
|---|---|
| Gerät | : Agilent 7890 A |
| Säule | : 30 m BGB-174 S, Innendurchmesser 0,25 mm, Filmdicke 0,25 μm |
| Eluent | : Helium |
| Detektor | : FID, 250 °C |
| Flussrate | : 1,5 mL/min (Fluss geregelt) |
| Injektion | : 0,2 μL, Split 100:1 |
| Temperatur | : Start 60 °C, 2 °C/min auf 80 °C, 30 min isotherm bei 80 °C, 10 °C/min auf 210 °C |
| Laufzeit | : 53 min |

### B) Herstellungbeispiele

**<u>Beispiel 1:</u> Umsetzung von Isopulegol**

Der Gasphasenreaktor wurde mit X540T 1/8 (150 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Im Folgenden wurden Verdampfer und Reaktor bei einer Temperatur von 170 °C betrieben und bei Normaldruck von Stickstoff (20 NL/h) durchströmt. Isopulegol (Wassergehalt 3.7 Gew.-%, 15 g/h, 97.2 mmol/h) wurde kontinuierlich in den Verdampfer eingebracht. Das Produktgemisch wurde am Reaktorausgang kondensiert und die Zusammensetzung gaschromatographisch analysiert. Nach jeweils fünf Stunden Versuchsdauer wurden Reaktor und Verdampfer unter Stickstoffstrom (20 NL/h) abgekühlt und der Versuch nach 18 h ohne Katalysatorwechsel fortgesetzt.

Der Umsatz von Isopulegol war über die gesamte Reaktionsdauer vollständig.

**Tabelle 1: Umsatz, Massenbilanz und Produktzusammensetzung bei der Umsetzung von Isopulegol.**

| Versuch | Zeit [h] | Umsatz Isopulegol [%]* | Selektivität Menthon [%]* | Selektivität Isomenthon [%]* | Selektivität Menthone (gesamt) [%]* | Massenbilanz [%] |
|---|---|---|---|---|---|---|
| 1 | 5 | 100 | 50,4 | 25,6 | 76,0 | 95 |
| 2 | 5 | 100 | 56,6 | 28,0 | 84,6 | > 98 |
| 3 | 5 | 100 | 59,2 | 29,3 | 88,5 | 98 |
| *Angaben in basierend auf Flächenprozent. | | | | | | |

Der Umsatz von Isopulegol zu Menthon erfolgt in guten bis sehr guten Ausbeuten bis zu 88,5%. Das Verhältnis von Menthon zu Isomenthon liegt für alle Versuche im Bereich 65/35 bis 70/30 (Menthon/Isomenthon) (siehe Tabelle 1).

**<u>Beispiel 2:</u> Umsetzung von enantiomerenangereichertem Isopulegol (ee > 99%); Aktivierung des Katalysators mit Citronellol**

Der Gasphasenreaktor wurde mit X540T 1/8 (150 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20-40 NL/h) bei einer Temperatur von 170-180 °C aktiviert. Im Folgenden wurden Verdampfer und Reaktor bei einer Temperatur von 170 °C betrieben und bei Normaldruck von Stickstoff (20 NL/h) durchströmt. Isopulegol (ee > 99%, Wassergehalt 3.7 Gew.-%, 15 g/h, 97.2 mmol/h) wurde kontinuierlich in den Verdampfer eingebracht. Das Produktgemisch wurde am Reaktorausgang kondensiert und die Zusammensetzung gaschromatographisch analysiert. Nach jeweils fünf Stunden Versuchsdauer wurden Reaktor und Verdampfer unter Stickstoffstrom (20 NL/h) abgekühlt und der Versuch nach 18 h ohne Katalysatorwechsel fortgesetzt. Zwischen Versuch 6 und 7 wurde der Reaktor für fünf Stunden bei 170 °C mit Citronellol (15 g/h, 96.0 mmol/h) durchströmt.

**Tabelle 2: Übersicht über die Produktzusammensetzung bei der Umsetzung von Isopulegol (ee > 99%).**

| Versuch | Zeit | Umsatz Isopulegol | Menthon | | Isomenthon | | Selektivität Menthone (gesamt) | Massenbilanz |
|---|---|---|---|---|---|---|---|---|
| | | | Selektivität | *ee* | Selektivität | *ee* | | |
| | [h] | [%]* | [%]* | [%] | [%]* | [%] | [%]* | [%] |
| 1 | 5 | 100 | 50,3 | 65,8 | 25,1 | 67,4 | 75,4 | 95 |
| 2 | 5 | 100 | 55,0 | 75,5 | 26,3 | 77,8 | 81,3 | > 98 |
| 3 | 5 | 100 | 58,4 | 84,7 | 28,1 | 85,8 | 86,5 | > 98 |
| 4 | 5 | 100 | 58,5 | 85,1 | 28,7 | 86,3 | 87,2 | 98 |
| 5 | 5 | 99,5 | 58,3 | 85,8 | 29,1 | 87,4 | 87,4 | > 98 |
| 6 | 5 | 97,7 | 58,3 | 89,2 | 29,7 | 90,2 | 88,0 | > 98 |
| 7 | 5 | 51,5 | 42,9 | > 99 | 24,5 | > 99 | 67,4 | > 98 |
| 8 | 5 | 35,2 | 34,4 | > 99 | 19,9 | > 99 | 54,3 | > 98 |
| *Angaben basierend auf Flächenprozent. | | | | | | | | |

Die Umsätze von Isopulegol zu Menthon (gesamt) steigen bis einschließlich Versuch 6 sukzessive bis auf einen Wert von 88% an. Ähnlich verhält es sich mit den Enatiomerenüberschüssen: Ausgehend von mäßigen Enantiomerenüberschüssen für Versuch 1 ist in der Folge eine Steigerung auf bis zu 90% ee bei Versuch 6 zu beobachten. Nach der Aktivierung des Katalysators durch Citronellol geht der Umsatz zwar deutlich zurück (bis zu 65% an Ausgangsmaterial werden nicht umgesetzt). Der Enantiomerenüberschuss jedoch wird in den Versuchen 7 und 8 überraschenderweise auf größer als 99% gesteigert. (siehe Tabelle 2).

**Beispiel 3: Umsetzung von enantiomerenangereichertem Isopulegol (ee > 99%); Aktivierung des Katalysators mit Citronellol**

Der Gasphasenreaktor wurde mit X540T 1/8 (150 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-Gasstrom (20 - 40 NL/h) bei einer Temperatur von 170-180 °C aktiviert. Im Folgenden wurden Verdampfer und Reaktor bei einer Temperatur von 170 °C betrieben und bei Normaldruck von Stickstoff (20 NL/h) durchströmt. Isopulegol (ee > 99%, Wassergehalt 3.7 Gew.-%, 15 g/h, 97.2 mmol/h) wurde kontinuierlich in den Verdampfer eingebracht. Das Produktgemisch wurde am Reaktorausgang kondensiert und die Zusammensetzung gaschromatographisch analysiert. Nach jeweils fünf Stunden Versuchsdauer wurden Reaktor und Verdampfer unter Stickstoffstrom (20 NL/h) abgekühlt und der Versuch nach 18 h ohne Katalysatorwechsel fortgesetzt. Zwischen Versuch 1 und 2 wurde der Reaktor für fünf Stunden bei 170 °C mit Citronellol (15 g/h, 96.0 mmol/h) durchströmt. Vor Versuch 11 erfolgte eine Temperaturerhöhung auf 180 °C.

**Tabelle 3: Übersicht über die Produktzusammensetzung bei der Umsetzung von Isopulegol (ee > 99%).**

| Versuch | Zeit [h] | Umsatz Isopulegol | Menthon | | Isomenthon | | Selektivität Menthone (gesamt) | Massenbilanz |
|---|---|---|---|---|---|---|---|---|
| | | | Selektivität | *ee* | Selektivität | *ee* | | |
| | | [%]* | [%]* | [%] | [%]* | [%] | [%]* | [%] |
| 1 | 5 | 100 | 52,8 | 71,0 | 25,8 | 71,3 | 78,6 | 94 |
| 2 | 5 | 92,8 | 58,3 | 98,3 | 30,1 | 98,6 | 88,4 | > 98 |
| 3 | 5 | 92,3 | 58,7 | 98,3 | 30,6 | 98,6 | 89,3 | > 98 |
| 4 | 5 | 90,0 | 58,4 | 98,5 | 30,6 | 98,4 | 89,0 | > 98 |
| 5 | 5 | 89,4 | 58,4 | 98,3 | 30,7 | >99 | 89,1 | > 98 |
| 6 | 5 | 87,6 | 57,9 | 98,5 | 30,4 | 98,6 | 88,3 | > 98 |
| 7 | 5 | 86,6 | 57,7 | 98,6 | 30,3 | 98,7 | 88,0 | > 98 |
| 8 | 5 | 84,1 | 57,1 | 98,8 | 30,0 | > 99 | 87,1 | > 98 |
| 9 | 5 | 82,6 | 56,5 | 98,9 | 29,7 | > 99 | 86,2 | > 98 |
| 10 | 5 | 78,8 | 55,5 | 99,0 | 29,2 | > 99 | 84,7 | > 98 |
| 11** | 5 | 89,0 | 57,1 | 98,2 | 30,2 | > 99 | 87,3 | > 98 |
| 12** | 5 | 87,7 | 56,6 | 98,3 | 30,0 | 98,6 | 86,6 | > 98 |
| *Angaben basierend auf Flächenprozent; **Reaktionstemperatur 180 °C | | | | | | | | |

Durch Aktivierung des Katalysators mit Citronellol konnten auch in diesem Fall die Enantiomerenüberschüsse von Versuch 2 an auf größer als 98% erhöht werden. Der Umsatzrückgang auf unter 80% in Versuch 10 konnte überraschenderweise durch eine mäßige Temperaturerhöhung um 10 °C kompensiert werden. Hohe Enantiomerenüberschüsse werden also nicht zwingend auf Kosten des Umsatzes erreicht. (siehe Tabelle 3).

**Beispiel 4: Umsetzung von enantiomerenangereichertem Isopulegol (ee > 99%); Aktivierung des Katalysators mit 1-Nonanol**

Der Gasphasenreaktor wurde mit X540T 1/8 (75 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Anschließend wurde der Reaktor von 1-Nonanol (7.5 g/h, 52.0 mmol/h) durchströmt. Verdampfer und Reaktor wurden im Folgenden bei einer Temperatur von 170 °C betrieben und bei Normaldruck von Stickstoff (20 NL/h) durchströmt. Isopulegol (ee > 99%, Wassergehalt 3.7 Gew.-%, 7.5 g/h, 48.6 mmol/h) wurde kontinuierlich in den Verdampfer eingebracht. Das Produktgemisch wurde am Reaktorausgang kondensiert und die Zusammensetzung gaschromatographisch analysiert.

**Tabelle 4: Übersicht über die Produktzusammensetzung bei der Umsetzung von Isopulegol (ee > 99%) bei Aktivierung des Katalysators mit 1-Nonanol.**

| Versuch | Zeit [h] | Umsatz Isopulegol [%]* | Menthon Selektivität [%]* | Menthon *ee* [%] | Isomenthon Selektivität [%]* | Isomenthon *ee* [%] | Selektivität Menthone (gesamt) [%]* | Massenbilanz [%] |
|---|---|---|---|---|---|---|---|---|
| 1 | 5 | 96,8 | 56,8 | 98,2 | 32,1 | 98,4 | 88,9 | > 98 |
| *Angaben basierend auf Flächenprozent. | | | | | | | | |

Die Behandlung des mit Wasserstoff aktivierten Katalysators mit 1-Nonanol führt bei hohen Umsätzen zu Menthon in enatiomerenangereicherter Form. Überraschenderweise können auch für die Verwendung dieses Alkohols Enantiomerenüberschüsse von über 98% erreicht werden (siehe Tabelle 4).

**Beispiel 5: Umsetzung von enantiomerenangereichertem Isopulegol (ee > 99%); Aktivierung des Katalysators mit 1,4-Butandiol**

Der Gasphasenreaktor wurde mit X540T 1/8 (75 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Anschließend wurde der Reaktor von 1,4-Butandiol (7.5 g/h, 83.2 mmol/h) durchströmt. Verdampfer und Reaktor wurden im Folgenden bei einer Temperatur von 170 °C betrieben und bei Normaldruck von Stickstoff (20 NL/h) durchströmt. Isopulegol (ee > 99%, Wassergehalt 3.7 Gew.-%, 7.5 g/h, 48.6 mmol/h) wurde kontinuierlich in den Verdampfer eingebracht. Das Produktgemisch wurde am Reaktorausgang kondensiert und die Zusammensetzung über gaschromatographische Untersuchungen analysiert.

**Tabelle 5: Übersicht über die Produktzusammensetzung bei der Umsetzung von Isopulegol (ee > 99%) bei Aktivierung des Katalysators mit 1,4-Butandiol.**

| Versuch | Zeit [h] | Umsatz Isopulegol [%]* | Menthon Selektivität [%]* | Menthon *ee* [%] | Isomenthon Selektivität [%]* | Isomenthon *ee* [%] | Selektivität Menthone (gesamt) [%]* | Massenbilanz [%] |
|---|---|---|---|---|---|---|---|---|
| 1 | 5 | 96,1 | 56,5 | 93,0 | 28,8 | 93,4 | 85,3 | > 98 |
| *Angaben basierend auf Flächenprozent. | | | | | | | | |

Die Behandlung des mit Wasserstoff aktivierten Katalysators mit 1,4-Butandiol führt ebenfalls zu guten Umsätzen von Isopulegol zu Menthon. Die Enantiomerenüberschüsse bleiben jedoch geringfügig hinter den für 1-Nonanol und Citronellol erreichten Ergebnissen zurück. Die Eignung von Diolen für diese Umsetzung konnte jedoch erfolgreich demonstriert werden (siehe Tabelle 5).

Auf die Offenbarung hierin zitierter Dokumente wird in vollem Umfang Bezug genommen.

## Patentansprüche

1. Verfahren zur Umsetzung von Isopulegol, wobei Isopulegol in der Gasphase mit einem aktivierten Kupferkatalysator in Kontakt gebracht wird und gegebenenfalls nach der Umsetzung ein Menthon-haltiges Reaktionsprodukt isoliert wird.

2. Verfahren nach Anspruch 1, wobei der Kupferkatalysator mit Wasserstoff oder Wasserstoff und einem Alkohol aktiviert wird.

3. Verfahren nach einem der vorhergehenden Ansprüche, wobei das eingesetzte Isopulegol ein Enantiomer der Formel I umfasst.

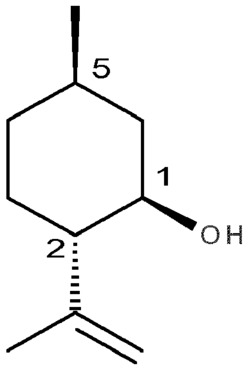

I

4. Verfahren nach einem der vorhergehenden Ansprüche, wobei die Reaktion bei einer Temperatur von 150-250 °C, insbesondere 160-200 °C, durchgeführt wird.

5. Verfahren nach einem der vorhergehenden Ansprüche, wobei der Kupferkatalysator neben Kupfer wenigstens ein weiteres Elemente ausgewählt unter Aluminium, Mangan, Barium, Chrom, Calcium, Eisen und Sauerstoff enthält.

6. Verfahren nach Anspruch 5, wobei der Kupferkatalysator Kupfer und wenigstens ein weiteres Element ausgewählt unter Aluminium, Mangan, Barium, Chrom, Calcium und Eisen elementar und/oder als Oxide beinhaltet.

7. Verfahren nach Anspruch 6, wobei die Oxide als Einelementoxide, Doppeloxide und/oder Mehrfachoxide vorliegen.

8. Verfahren nach einem der vorhergehenden Ansprüche, wobei der Kupferkatalysator einen Anteil von mindestens 20% bis zu 100% Kupferverbindung bezogen auf die Trockenmasse des Katalysators aufweist.

9. Verfahren nach einem der vorhergehenden Ansprüche, wobei der Katalysator 30-40% Kupferoxid, 10-25% Aluminiumoxid, 10-25% Manganoxid und 30-40% Aluminiumkupferoxid umfasst.

10. Verfahren nach einem der vorhergehenden Ansprüche, wobei der Katalysator vor der Umsetzung mit Wasserstoff oder Wasserstoff und einem Alkohol gleichzeitig oder zeitlich versetzt in beliebiger Reihenfolge, gegebenenfalls in einem Trägergasstrom, aktiviert wird.

11. Verfahren nach Anspruch 10, wobei das Trägergas Stickstoff oder Argon ist und gegebenenfalls bis zu 10 Vol.-% Wasserstoff enthält.

12. Verfahren nach einem der vorhergehenden Ansprüche, wobei der für die Aktivierung verwendete Alkohol ausgewählt ist unter gesättigten oder ein- oder mehrfach ungesättigten, geradkettigen oder verzweigten oder cyclischen aliphatischen oder aromatischen Mono- oder Polyolen, insbesondere Mono- oder Diolen, oder Kombinationen davon.

13. Verfahren nach Anspruch 12, wobei der Alkohol ein $C_1$-$C_{20}$,-, $C_3$-$C_{16}$- oder $C_4$-$C_{12}$-Alkohol ist.

14. Verfahren nach einem der vorhergehenden Ansprüche, wobei das Umsetzungsprodukt Menthon und/oder Isomenthon mit einem erhöhten Anteil des Enantiomers mit (*R*)-Konfiguration in Position 5 umfasst.

15. Verfahren nach einem der vorhergehenden Ansprüche, wobei im Umsetzungsprodukt Menthon und/oder Isomenthon im Wesentlichen enantiomerenrein vorliegt.

16. Verwendung eines nach einem der vorhergehenden Ansprüche hergestellten Reaktionsprodukts als Zusatz in Lebensmitteln, Kosmetika, pharmazeutischen Erzeugnissen, Tabakformulierungen, Haushaltsprodukten und Wäschepflegeprodukten.

Europäisches Patentamt
European Patent Office
Office européen des brevets

# EUROPÄISCHER RECHERCHENBERICHT

Nummer der Anmeldung
EP 14 15 8373

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | TREIBS W ET AL: "ZUR KATALYTISCHEN DEHYDRIERUNG HYDRO-AROMATISCHER VERBINDUNGEN", CHEMISCHE BERICHTE, VCH, DE, Bd. 60, 1. Januar 1927 (1927-01-01), Seiten 2335-2341, XP008048012, ISSN: 0009-2940<br>* Seite 2338, Absatz 2 *<br>* Seite 2339, Absatz 3 *<br>* Seite 2336, letzter Absatz *<br>----- | 1-15 | INV.<br>C07C45/29<br>C07C49/407<br>A61Q11/00<br>A61K8/35 |
| E | EP 2 706 054 A1 (BASF SE [DE]) 12. März 2014 (2014-03-12)<br>* Ansprüche 12,13 *<br>----- | 16 | |
| X | WO 2008/016855 A1 (WRIGLEY W M JUN CO [US]; BAZEMORE RUSSELL A [US]; HARRISON CHARLES J [) 7. Februar 2008 (2008-02-07)<br>* Ansprüche *<br>----- | 16 | |
| A | US 2006/160719 A1 (EMURA MAKOTO [JP] ET AL) 20. Juli 2006 (2006-07-20)<br>* Seite 5, rechte Spalte, Absatz 2 *<br>----- | 1-15 | RECHERCHIERTE SACHGEBIETE (IPC) |
| A | MARÍA ELENA GONZÁLEZ-NÚÑEZ ET AL: "Oxidation of Alcohols to Carbonyl Compounds with CrO 3 .SiO 2 in Supercritical Carbon Dioxide", THE JOURNAL OF ORGANIC CHEMISTRY, Bd. 71, Nr. 3, 1. Februar 2006 (2006-02-01), Seiten 1039-1042, XP055113312, ISSN: 0022-3263, DOI: 10.1021/jo052137j<br>* Tabelle 1 *<br>----- | 1-15 | C07C<br>A61K<br>A61Q |
| A,D | DE 42 36 111 A1 (HENKEL KGAA [DE]) 28. April 1994 (1994-04-28)<br>* das ganze Dokument *<br>----- | 1-15 | |

Der vorliegende Recherchenbericht wurde für alle Patentansprüche erstellt

1

| Recherchenort | Abschlußdatum der Recherche | Prüfer |
|---|---|---|
| München | 11. April 2014 | Österle, Carmen |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 14 15 8373

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-04-2014

| Im Recherchenbericht angeführtes Patentdokument | Datum der Veröffentlichung | Mitglied(er) der Patentfamilie | Datum der Veröffentlichung |
|---|---|---|---|
| EP 2706054 A1 | 12-03-2014 | EP 2706054 A1 | 12-03-2014 |
| | | WO 2014037264 A1 | 13-03-2014 |
| WO 2008016855 A1 | 07-02-2008 | CA 2659398 A1 | 07-02-2008 |
| | | CN 101522159 A | 02-09-2009 |
| | | EP 2046274 A1 | 15-04-2009 |
| | | RU 2009104151 A | 10-09-2010 |
| | | US 2009317461 A1 | 24-12-2009 |
| | | WO 2008016855 A1 | 07-02-2008 |
| US 2006160719 A1 | 20-07-2006 | KEINE | |
| DE 4236111 A1 | 28-04-1994 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 82180463 B
- DE 4236111 A1
- US 4134919 A


### In der Beschreibung aufgeführte Nicht-Patentliteratur


- *Spec. Chem.,* 1987, 193
- *Acta Chem. Scand. B,* 1979, 148
- *J. Org. Chem.,* 1980, 2030
- *Tetrahedron,* 1979, 1789
- **W. TREIBS et al.** *Chem. Ber.,* 1927, 2335
- **FORTI et al.** *Synthesis,* 2001, vol. 1, 52